(19) Europäisches Patentamt
European Patent Office
Office européen des brevets

(11) Veröffentlichungsnummer : **0 273 957 B1**

(12) # EUROPÄISCHE PATENTSCHRIFT

(45) Veröffentlichungstag der Patentschrift : **25.09.91 Patentblatt 91/39**

(51) Int. Cl.$^5$ : **G01N 9/32,** G01N 11/06, G01N 33/22

(21) Anmeldenummer : **87904528.4**

(22) Anmeldetag : **13.07.87**

(86) Internationale Anmeldenummer : **PCT/EP87/00377**

(87) Internationale Veröffentlichungsnummer : **WO 88/00693 28.01.88 Gazette 88/03**

(54) **VERFAHREN UND VORRICHTUNG ZUM MESSEN VON GASEIGENSCHAFTEN.**

(30) Priorität : **14.07.86 DE 3623664**

(43) Veröffentlichungstag der Anmeldung : **13.07.88 Patentblatt 88/28**

(45) Bekanntmachung des Hinweises auf die Patenterteilung : **25.09.91 Patentblatt 91/39**

(84) Benannte Vertragsstaaten : **AT BE CH DE FR GB IT LI LU NL SE**

(56) Entgegenhaltungen :
**EP-A- 0 022 493**
**EP-A- 0 151 486**
**DE-A- 3 237 130**
**US-A- 1 958 878**
**US-A- 2 783 641**

(73) Patentinhaber : **Ruhrgas Aktiengesellschaft**
**Huttropstrasse 60 Postfach 10 32 52**
**W-4300 Essen 1 (DE)**

(72) Erfinder : **ALTEMARK, Detlef**
**Am Fuchspass 20**
**W-4270 Dorsten (DE)**
Erfinder : **HESS, Robert**
**Gerschermann Weg 12**
**W-4300 Essen (DE)**
Erfinder : **HILLRINGHAUS, Karl-Heinz**
**Birkenstrasse 70**
**W-4250 Bottrop (DE)**

(74) Vertreter : **Zenz, Joachim Klaus et al**
**Am Ruhrstein 1**
**W-4300 Essen 1 (DE)**

**EP 0 273 957 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezeiht sich einverfahren bzw. eine Vorrichtung zum Messen von Gaseigenschaften nach dem Oberbegriff des Patentanspruchs 1 bzw. des Patentanspruchs 14.

Aus der EP-OS 0 022 493 sind ein Verfahren und eine Vorrichtung zur verbrennungslosen Messung und-/oder Regelung der Wärmemengenzufuhr zu Gasverbrauchseinrichtungen bekannt. Danach ist es möglich, neben der Dichte und der dynamischen Viskosität auch andere Eigenschaften von Brenngasen zu bestimmen, wie den Brennwert, den Heizwert oder den Wobbe-Index. Dabei wird wenigstens ein Teil des zu analysierenden Gases zur Messung der dynamischen Vikosität durch einen Laminarwiderstand in Form einer kapillare und zur Messung der Dichte durch einen Turbulenzwiderstand in Form einer Lochblende geleitet. Soweit zwecks Bestimmung des Brenn- oder Heizwertes sowohl die dynamische Viskosität als auch die Dichte gemessen werden müssen, werden der Laminarwiderstand und der Turbulenzwiderstand in Reihe hintereinander angeordnet. Zur Bestimmung der dynamischen Viskosität und der Dichte werden zumindest zwei Meßwerte benötigt, nämlich die Druckdifferenz über dem betreffenden Strömungswiderstand sowie der Volumenstrom (Volumen/Zeit) des den Strömungswiderstand passierenden Gases. Die Messungen erfolgen im kontinuierlichen Gasstrom, so daß die Gasdrücke jeweils vor und hinter dem Strömungswiderstand gemessen werden; somit sind für die Messung der dynamischen Viskosität und der Dichte des Gases insgesamt fünf Meßwerte zu erfassen und zu verknüpfen. Außerdem muß das permanent strömende Gas der Hauptleitung oder einem Verbraucher zugeführt werden.

Aus der US-A-2 783 641 sind ferner Verfahren und Vorrichtung der gattungsgemäßen Art bekannt, bei denen die Druckdifferenz am Sensor durch das System vorgegeben ist (isobar arbeitendes Volumenverdrängungssystem). Der Volumenstrom kann bei vorgegebener Wegänderung durch Zeitmessung bestimmt werden. Dadurch kann die Messung der Dichte und dynamischen Viskosität auf jeweils eine Zeitmessung reduziert werden. Bisher war es mit vertretbarem Aufwand nicht möglich, die Abdichtung des Meßgasvolumens gegenüber der Umgebung und eine reibungsfreie Führung des Volumenverdrängungsbauteils während der Messung zu gewährleisten. Daher waren unkontrollierte Druckänderungen in der Regel unvermeidbar. Besonders nachteilig wirken sich auch Materialalterungen insbesondere beim Membransystem aus. Auch Restvolumina nach der Entspannung, verbunden mit unerwünschten Vermischungseffekten in dem Meßsystem konnten nicht zuverlässig vermieden werden.

Die EP-A-0 151 486 beschreibt ein anderes, nämlich ein isochores Meßprinzip. Bei der dort beschriebenen isochoren Entspannung eines Druckbehälters können aus dem zeitlichen Verlauf des Behälterdruckes Informationen über die Stoffgrößen $\rho$ und $\eta$ gewonnnen werden. Um jedoch für die Erfassung beider Stoffgrößen in den Sensoren reproduzierbare und stabile Strömungsbedingungen zu erreichen, müssen diese unter bestimmten Randbedingungen ausreichend lange durchströmt werden. Dies kann bei einem sinnvollen Volumen des Druckbehälters nur durch einen hohen Behälterdruck erreicht werden. Hohe Behälterdrücke haben jedoch den Nachteil stark instationärer Strömungsverhältnisse in den Sensoren. Außerdem werden die Meßsignale durch zusätzliche Gaseigenschaften, z.B. die Kompressibilität, beeinflußt. Das große Volumen hat außerdem eine unzulässig hohe Trägheit des Meßsystems bei Änderungen in den Stoffgrößen zur Folge, da durch Vermischung in der Meßkammer eine sehr starke Zeitverzögerung der Meßsignaländerung bei Stoffgrößenänderungen unvermeidbar ist.

Der Erfindung liegt die Aufgabe zugrunde, die oben erwähnten Nachteile sowohl des isobaren als auch des isochoren Verfahrens weitgehend zu vermeiden und bei vergleichsweise geringem baulichen und betrieblichen Aufwand ein schnell anzeigendes und präzises Meßsystem zum Messen von Gaseigenschaften, insbesondere der dynamischen Viskosität und/oder der Dichte zur Verfügung zu stellen.

Diese Aufgabe wird verfahrensmäßig erfindungsgemäß dadurch gelöst, daß ein Gasspeicherbehälter verwendet wird, der in vorgegebener Abhängigkeit von Druckdifferenzen zwischen seinem Innenraum und seiner Umgebungsatmosphäre Volumenänderungen erfährt, daß die genannte vorgegebene Abhängigkeit des Gasspeicherbehälters bestimmt wird, daß danach der Meßzyklus unter Füllen des Gasspeicherbehälters mit dem zu untersuchenden Gas begonnen wird, wobei das Volumen des Gasspeicherbehälters und die Druckdifferenz nach der zuvor bestimmten Abhängigkeit vergrößert werden, daß während des Abströmens des Gases sowohl das Volumen des Gasspeicherbehälters als auch die Druckdifferenz nach der vorgegebenen Abhängigkeit verringert und die Zeit für eine vorgegebene Volumenverringerung oder eine vorgegebene Druckdifferenzverringerung gemessen wird und daß der Volumenstrom und die gesuchte Gaseigenschaft aus der gemessenen Zeit und wahlweise der genannten Volumenverringerung oder der zugehörigen Druckdifferenzverringerung bestimmt werden.

Ausgehend von der gattungsgemäßen Vorrichtung ist erfindungsgemäß dadurch gekennzeichnet, daß der Gasspeicherbehälter so ausgebildet ist, daß sein Innenraum in vorgegebener Abhängigkeit von Druckdifferenzen zur Umgebungsatmosphäre Volumenänderungen erfährt, daß die Zeitmeßvorrichtung wahlweise in

Abhängigkeit von vorgegebenen Volumina des Gasspeicherbehälters oder von Druckdifferenzen ein- und ausschaltbar ist; und daß Mittel zur Bestimmung einer Abhängigkeit von Druckdifferenzen zwischen dem Gasspeicherbehälter-Innenraum und der Umgebungsatmosphäre zu dabei auftretenden Volumenänderungen des Gasspeicherbehälters vorgesehen sind.

Die Erfindung basiert demnach auf dem Grundgedanken, eine nicht-isochore und nicht-isobare Messung durchzuführen bzw. ein entsprechendes Meßgerät vorzusehen. Die Messung wird diskontinuierlich durchgeführt. Durch Kalibrierung wird zunächst die Arbeitskennlinie eines aktiven Volumenstrom-Dosierelements bestimmt und die Zeit, die ein bestimmtes Gasvolumen zum Passieren des dem aktiven Volumenstrom-Dosierelements nachgeschalteten Strömungswiderstandes benötigt, gemessen. Aus dieser Zeitmessung und der Arbeitskennlinie wird dann die zu messende Größe errechnet.

Durch die Erfindung wird es möglich, die Messung der Dichte, der dynamischen Viskosität und bestimmter mit diesen Größen korrelierter Eigenschaften von Gasen, insbesondere Brenngasen, auf eine einfache Zeitmessung zu reduzieren. Hierzu ist weder eine aufwendige Vorrichtung zum Konstanthalten des Volumenstromes oder der Druckdifferenzen über den Strömungswiderständen erforderlich, noch bedarf es weiterer Meßwerte. Allenfalls werden für etwaige Korrelationen die thermodynamischen Zustandsgrößen P und T erfaßt. Das Bauvolumen der Meßvorrichtung kann vergleichsweise klein gehalten werden. Es sind nur kleine Gasvolumina in der Größenordnung einiger $cm^3$ erforderlich, so daß die Meßzeiten unterhalb einer Sekunde liegen können und die für die Messung abgezweigte Gasmenge nicht in die Hauptgasleitung zurückgeführt zu werden braucht.

Bei einer bevorzugten Ausführungsform der Erfindung ist der das Volumenstrom-Dosierelement bildende Gasspeicherbehälter eine sogenannte kapselfeder; diese zeichnet sich durch ein besonders gut reproduzierbares elastomechanischen Verhalten aus und ermöglicht die problemlose und genaue Zuordnung von Volumenänderungen und Druckdifferenzen zu einem bestimmten Volumenstrom V.

In alternativer Ausführungsform kann der Gasspeicherbehälter auch als federbelasteter Balg, federbelastete Kolben-Zylindervorrichtung oder als anderes Ausdehnungsgefäß ausgebildet sein, dessen Volumenänderung durch einen geeigneten Wegaufnehmer erfaßt werden kann bzw. dessen in geeigneter Weise erfaßte Differenzdruckänderung sich reprodzierbar einem bestimmten Volumenstrom zuordnen läßt.

Eine wesentliche Erhöhung der Meßgenauigkeit läßt sich sowohl bei der Dichtemessung als auch bei der Viskositätsmessung in Weiterbildung des erfindungsgemäßen Verfahrens dadurch erreichen, daß zu untersuchendes Gas aus dem Gasspeicherbehälter in zeitlich versetzten Meßphasen einem Dichtesensor (Blende) und einem Viskositätssensor (Kapillare) zugeführt wird und für die Dichte und die Viskosität repräsentative Meßwerte aufgenommen werden, wobei ein begrenzter Viskositätseinfluß auf den Dichtesensor und ein begrenzter Dichteeinfluß auf den Viskositätssensor zugelassen werden, und daß die aus diesen Einflüssen resultierenden Meßfehler der aufgenommenen Meßwerte rechnerisch korrigiert werden. Der vorzugsweise als Blende ausgebildete Dichtesensor braucht bei dieser Weiterbildung des erfindungsgemäßen Verfahrens nicht mehr so exakt ausgelegt zu werden, daß mit der Blende selektiv die Dichte gemessen wird. Ebensowenig braucht der als Kapillare ausgebildete Viskositätssensor so exakt ausgelegt zu werden, daß selektiv die Viskosität gemessen wird. Es kann beispielsweise eine kürzere Kapillarenlänge zugelassen werden. Mit Hilfe der ohnehin bei Meßverfahren und Meßvorrichtungen der erfindungsgemäßen Art eingesetzten Mikroprozessoren und Speicher können sehr genaue Meßergebnisse erzielt und die systematischen Meßfehler weitgehend eliminiert werden.

Die rechnerische Korrektur erfolgt vorzugsweise iterativ. Zu diesem Zweck wird jeder Meßwert zunächst als näherungsweiser Startwert für einen ersten Iterationsschritt benutzt und aus dem Iterationsergebnis ein korrigierter Startwert für einen zweiten Iterationsschritt abgeleitet. Die Iteration wird danach solange fortgesetzt, bis die relative Abweichung zwischen aufeinanderfolgenden Iterationsschritten (N und N-1) kleiner als ein vorgegebener und gespeicherter Genauigkeitsgrenzwert ist.

Zweckmäßige Ausgestaltungen des Erfindungsgegenstandes, die insbesondere eine exakte und automatisch wiederholt durchführbare Messung und Meßwertauswertung gewährleisten, sind in weiteren Ansprüchen enthalten.

Die vorgenannten, erfindungsgemäß zu verwendenden Bauteile bzw. Verfahrensschritte unterliegen in ihrer Größe, Formgestaltung, Materialauswahl und technischen Konzeption bzw. ihren Verfahrensbedingungen keinen besonderen Ausnahmebedingungen, so daß die in dem jeweiligen Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der zugehörigen Zeichnung, in der eine bevorzugte Ausführungsform der erfindungsgemäßen Meßvorrichtung dargestellt ist. In der Zeichnung zeigen:

Fig. 1 eine schematische Ansicht einer Meßvorrichtung zum Messen der Dichte und dynamischen Viskosität und damit korrelierter Eigenschaften von Gas in schematischer Darstellung;

Fig. 2 für die Meßvorrichtung gemäß Fig. 1 ein Weg/Zeit-Diagramm mit Schaltpunkten für die Zeitmessung über einen vollen Meßzyklus; und

Fig. 3 ein ähnliches Weg/Zeit-Diagramm des Zeitverhaltens einer in der Meßvorrichtung gemäß Fig. 1 dargestellten Kapselfeder bei Verwendung als Gasspeicherbehälter für verschiedene Gase.

Die Meßvorrichtung gemäß Figur 1 weist einen durch ein Ventil 1 absperrbaren Gaseinlaß 2 in Form einer von einer Gasversorgungsleitung 3 abzweigenden Gaszuführleitung auf. Der absperrbare Gaseinlaß 2 ist mit einem als Kapselfeder ausgebildeten Gasspeicherbehälter 4 über Rohrleitungen 5, 6 fluidisch verbunden.

Die Kapselfeder zeichnet sich durch ein sehr gut reproduzierbares Differenzdruck/Weg-Verhalten aus: Zwischen der Ausdehnung (Hub s) der Kapselfeder und der Druckdifferenz $\Delta p$ zwischen dem Innenraum 7 der Kapselfeder und der Umgebungsatmosphäre außerhalb der Kapselfeder besteht eine exakte Beziehung; der Hub s ist somit eine eindeutige Funktion der Druckdifferenz $\Delta p$. Aufgrund dieses funktionalen Zusammenhangs ist es möglich, die Druckdifferenzmessung zwischen dem Innenraum 7 des Gasspeicherbehälters 4 (Kapselfeder) und der Umgebungsatmosphäre durch eine Messung des Hubes s zu ersetzen, der auf den differenzdrucklosen Ruhezustand des Gasspeicherbehälters bezogen ist.

Die Kapselfeder 4 besteht aus einem flachen, geschlossenen Behälter, insbesondere aus Metall, dessen kreisrunde Boden- und Deckelflächen durch gewellte Membranen gebildet sind. Die Gaszuführung in den Innenraum 7 der Kapselfeder erfolgt bevorzugt über einen einzigen ortsfesten Anschluß in Form einer als Gaszuführ- und Gasabführleitung dienenden Rohrleitung 6, die in der Mitte einer Kapselmembran mündet. Hierdurch wird ein maximaler Hub s eines der ortsfesten Rohrleitung 6 diametral gegenüberliegenden Hubzeigers 8 entlang einer ortsfesten Hubskala 9 erreicht. Anstelle einer mechanischen, ablesbaren Hubanzeige kann auch eine elektrisch, optisch oder in sonstiger Weise abgreifbare Hubanzeige vorgesehen sein. Bei voreinstellbaren Hüben $s_1$, $s_2$ ... ist die Hubskala 9 mit Schaltpunkten 10 derart versehen, daß bei deren Erreichen durch den Hubzeiger 8 eine Zeitmessung durch eine zugeordnete Zeitmeßvorrichtung 11 begonnen bzw. beendet wird.

Über die Rohrleitung 6 und eine anschließende Rohrleitung 12 sowie davon abzweigende Rohrleitungen 13 und 14 ist der Gasspeicherbehälter 4 mit zwei Strömungswiderständen 15, 16 fluidisch verbunden. Diesen Strömungswiderständen sind Ventile 17, 18, insbesondere Absperrventile nachgeordnet, welche als absperrbarer Gasauslaß dienen, durch die das zu messende Gas ohne wesentlichen Druckverlust ausströmen kann, so daß es sich bereits unmittelbar hinter den Strömungswiderständen auf atmosphärischem Umgebungsdruck befindet. Bei dem Ausführungsbespiel gemäß Figur 1 befinden sich der Gasspeicherbehälter 4, die Leitungen 5, 6, 12 - 14, die Strömungswiderstände 15, 16 und die Ventile 1, 17, 18 innerhalb eines thermostatisierten Gehäuses 19. In dem Gehäuse 19 ist ein Temperaturfühler 20 für eine Temperaturregelung oder, falls die Thermostatisierung entfällt, für eine rechnerische Temperaturkompensation angeordnet. Ein Druckaufnehmer 21, der dem Temperaturfühler 20 eng benachbart im Gehäuse 19 angeordnet ist, mißt den Umgebungsdruck des Gasspeicherbehälters 4 und ermöglicht eine rechnerische Druckkompensation bei der Verarbeitung des Meßwertes.

Bei dem Strömungswiderstand 16 (Laminarwiderstand) handelt es sich um eine Kapillare mit bekannter Geometrie, für die die dynamische Viskosität $\eta$ bekanntlich proportional zum Druckabfall $\Delta p_l$ in der Kapillare und umgekehrt proportional zum Volumenstrom $\dot{V}$ durch die Kapillare ist, sofern die Strömung laminar ist (Hagen-Poiseuille-Gesetz).

$$\eta \sim \frac{\Delta p_\ell}{\dot{V}} \quad (1)$$

Der andere Strömungswiderstand (Turbulenzwiderstand) 15 ist eine dünnwandige Lochblende. Statt einer solchen Lochblende kann auch ein anderes als Turbulenzwiderstand dienendes Drosselorgan verwendet werden, für das die Dichte in einem begrenzten Reynolds-Zahlenbereich proportional zum Druckabfall $\Delta p_t$ und umgekehrt proportional zum Quadrat des Volumenstromes ($\dot{V}^2$) ist (Energieerhaltungsgesetz).

$$\rho \sim \frac{\Delta p_t}{\dot{V}^2} \quad (2)$$

Die erforderliche Messung von $\Delta p_l$ und $\dot{V}$ einerseits und von $\Delta p_t$ und $\dot{V}^2$ andererseits erfolgt erfindungsgemäß ausschließlich auf der Basis der Zeit, die ein bestimmtes Volumen des in dem Speicherbehälter 4 gespeicherten Gases bei geschlossenem Ventil 1 benötigt, um über einen der beiden Strömungswiderstände 15 oder 16 abzuströmen. Dieses Volumen wird durch den Hub s zwischen zwei Schaltpunkten 10 festgelegt. Diese Vorgehensweise ist möglich, weil für den Gasspeicherbehälter 4 eine eindeutige Abhängigkeit zwischen seinem Ausdehnungsvolumen V und der Druckdifferenz $\Delta p$ zwischen dem Innenraum 7 und der Umgebungsatmosphäre besteht: $V(\Delta p)$. Diese Abhängigkeit wird für den Gasspeicherbehälter innerhalb der Meßvorrichtung durch Kalibrierung mit einem geeigneten Eichgas bekannter Eigenschaften ermittelt und vorzugsweise in einem Rechengerät, z. B. einem Mikroprozessor abgespeichert, bevor der Meßzyklus beginnt.

Aus dem funktionalen Zusammenhang V (Δp) kann die zeitliche Änderung des Ausdehnungsvolumens V, nämlich der Volumenstrom $\dot{V}$, abgeleitet werden:

$$\dot{V} = \frac{dV}{d\Delta p} \cdot \frac{d\Delta p}{dt} \quad (3)$$

Darin ist dV/dΔp eine aus der vorerwähnten Kennlinie des Gasspeicherbehälters abgeleitete Funktion, die für die spezielle Bauart des Gasspeicherbehälters eine spezifische Größe ist.

Aus den Gleichungen (1) und (3) bzw. (2) und (3) erkennt man, daß der zeitliche Verlauf der Differenzdruckänderung: Δp(t) nur durch die dynamische Viskosität bestimmt wird (wenn ein Laminarwiderstand verwendet wird) bzw. nur durch die Dichte (wenn ein Turbulenzwiderstand verwendet wird).

In Figur 3 ist die zeitliche Änderung des Differenzdruckes Δp mit den Stoffgrößen η bzw. ρ als Parameter qualitativ dargestellt - und zwar für den Fall, daß der vollständig gefüllte Gasspeicherbehälter sich aufgrund seiner Federelastizität ohne Unterbrechung über einen der Strömungswiderstände 15 oder 16 entleert. Die Zeitdifferenz Δt bei einem Ausströmungsvorgang zwischen zwei vorgegebenen Hubmarkierungen $s_1$ und $s_2$ (entsprechend den Differenzdruckwerten $\Delta p_1$ und $\Delta p_2$) ist dann ein Maß für die dynamische Viskosität bzw. die Wurzel aus der Dichte des verwendeten Gases. Zwecks Erzielung möglichst exakter Meßergebnisse werden die Schaltpunkte, zwischen denen eine Zeitmessung erfolgt, so gewählt, daß der zeitliche Verlauf Δp(t) zwischen diesen beiden Schaltpunkten möglichst linear ist.

Wenn für die Ermittlung des Brennwertes $H_o$ von Gasen sowohl deren dynamische Viskosität als auch deren Dichte gemessen werden sollen, werden erfindungsgemäß die beiden Strömungswiderstände 15 und 16 während eines einzigen Meßzyklus bzw. während eines einzigen Ausströmungsvorganges, zeitlich nacheinander und getrennt voneinander mit dem zu untersuchenden Gas beaufschlagt. Bevorzugt wird zunächst die Dichte-Messung und erst nachfolgend die Messung der dynamischen Viskosität vorgenommen. Dadurch erfolgt die Dichte-Messung im Bereich relativ höherer Druckdifferenzen, so daß dabei eine möglichst kleine Änderung der Reynoldszahl während des Meßzeitraumes erreicht wird. Entsprechend wird dann die Messung der dynamischen Viskosität bei relativ kleinen Reynoldszahlen, also im Bereich relativ kleiner Druckdifferenzen vorgenommen. Durch das Messen beider Größen, sowohl der dynamischen Viskosität als auch der Dichte, während eines einzigen Ausströmvorganges des Gasspeicherbehälters, wird sichergestellt, daß beide Größen an demselben zuvor im Gasspeicherbehälter 4 gespeicherten Gas ermittelt werden.

Aus den beiden Meßwerten können dann in bekannter Weise, wie im einzelnen in der EP-OS 0 022 493 beschrieben, abhängige Größen, wie der Brennwert $H_o$ von Gasen nach einer Korrelationsfunktion, z. B. $H_o$ (η,ρ) ermittelt werden. Für die Ermittlung des Wobbe-Indexes $W_0$ ist in erster Näherung nur die Messung der dynamischen Viskosität erforderlich.

Um neben den Normwerten die Betriebwerte fürt η, ρ, $H_o$, $W_o$ ermitteln zu können, ist es notwendig, zusätzlich die Temperatur T und den Druck P des Meßgases an dessen Entnahmestelle (Temperatur- und Druckaufnahmer 26 in Gasversorgungsleitung 3) zu erfassen, um die gewünschte Umrechnung durchführen zu können.

Um die Meßgenauigkeit zu erhöhen, kann eine Kalibrierung der Meßsignale mittels eines Prüfgases, z.B. $CH_4$, in je nach Bedarf mehr oder weniger kurzen Zeitabständen erfolgen.

Der Fülldruck des Gaspeicherbehälters wird durch geeignetes Öffnen und Schließen des Ventils 1 begrenzt. Dies kann mit Hilfe eines entsprechend gewählten Schaltpunktes - Schaltpunkt $s_1$ gemäß Figuren 1 und 2 - an der Hub-Markierung 9 unter Zuhilfenahme einer Steuereinheit 22 erfolgen. Die Steuereinheit 22 ist entsprechend für die Betätigung aller Ventile, der Zeitmeßvorrichtung 11 sowie eines Mikroprozessors 23 in Verbindung mit dem Hub s des Gasspeicherbehälters 4 und den Schaltpunkten 10 vorgesehen. Die Steuereinheit 22 kann auch Bestandteil Rechengeräts bzw. Mikroprozessors 23 sein. Gleiches gilt für die Zeitmeßvorrichtung 11.

Die Strömungswiderstände 15 und 16 werden beim Füllen des Gasspeicherbehälters 4 ebenfalls mit dem Fülldruck beaufschlagt, um eine Beschleunigung des Meßvorganges zu erreichen. Der Fülldruck - und entsprechend der dadurch erzielte Differenzdruck - sollte möglichst klein sein, zum Beispiel nur einige mbar (hPa), um Kompressibilitätseffekte bei dem Ausströmvorgang auszuschließen und den Bereich kleiner Reynoldszahlen einhalten zu können.

Schließlich sollten die Schaltpunkte 10 derart gewählt werden, daß die Meßzeiten beim Durchströmen der Strömungswiderstände etwa gleich groß sind. Damit wird die Meßgenauigkeit der Zeitmessung für beide Meßgrößen gleich gewichtet.

Im übrigen werden die Schaltpunkte 10 so gewählt, daß die Gasströmung durch die Strömungswiderstände zunächst einen quasistationären Zustand erreicht, bevor die Zeitmessung ausgelöst wird. Dies ist im einzelnen aus Fig. 2 ersichtlich. Die sogenannten "Einlauf"-Zeiten werden also nicht in die Zeitmessung einbezogen, weil es sich bei dem erfindungsgemäßen Verfahren um ein diskontinuierliches Verfahren handelt, bei dem die Messung der Gaseigenschaften in jedem Meßzyklus an einem dosierten Gasvolumen durchgeführt wird.

Im folgenden werden das Verfahren zum Messen von Gaseigenschaften sowie die Funktionsweise der

Meßvorrichtung gemäß Figur 1 unter Bezugnahme auf Figur 2 beschrieben. Vor Beginn zumindest des ersten Meßzyklus wird die Meßvorrichtung und damit insbesondere die Charakteristik des als Kapselfeder ausgebildeten Gasspeicherbehälters 4 mit einem Prüfgas bekannter Eigenschaften kalibriert. Die entsprechenden Eichwerte sind oder werden im Rechengerät 23 gespeichert. Zu Beginn eines Arbeitszyklus sollte insbesondere der Gasspeicherbehälter 4 möglichst weitgehend von Restgasen aus vorhergehenden Arbeitszyklen (Meß-, Prüf- oder Spülgasen) frei sein. Zu diesem Zweck sollten die Membranen der dargestellten Kapselfeder 4 zur Minimierung des Innenraums 7 möglichst eng aufeinanderliegen und das Volumen der Anschlußleitung 6 durch kurze Leitungsbemessung minimiert sein. Zu Beginn des Meßzyklus ist der Differenzdruck im wesentlichen gleich Null. Nach Öffnen des Ventils 1 wird das zu untersuchende Gas über den Einlaß 2 in die Meßvorrichtung eingeleitet, bis der gewünschte Fülldruck erreicht ist, der Gasspeicherbehälter 4 also soweit ausgedehnt ist, daß der Hubzeiger 8 den Schaltpunkt $s_1$ erreicht. Die den Strömungswiderständen 15 und 16 zugeordneten Ventile 17 und 18 und das Ventil 24 sind dabei entweder geschlossen, damit sich der Solldruck rasch im Gasspeicherbehälter 4 aufbaut, oder zum Spülen wenigstens teilweise geöffnet. Danach wird zumindest das Zulaufventil 1 geschlossen (bei vorhergehender Öffnung werden auch die Ventile 18 und 24 geschlossen), und das Ventil 17 wird geöffnet, um das im Meßsystem, insbesondere im Gasspeicherbehälter 4 gespeicherte Gas über die Leitung 13 und den Strömungswiderstand 15 abfließen zu lassen, bis der Hubzeiger 8 den Schaltpunkt 3 erreicht hat. Wie aus Figur 2 zu sehen ist, beginnt die Zeitmessung, wenn der Hubzeiger 8 den Schaltpunkt $s_2$ erreicht hat, und sie endet, wenn der Hubzeiger 8 den Schaltpunkt $s_3$ erreicht hat. Am Schaltpunkt $s_3$ wird das Ventil 17 geschlossen und das Ventil 18 geöffnet, so daß danach das zu untersuchende Gas über die Kapillare 16 und das Ventil 18 abströmen kann. Sobald der Schaltpunkt $s_4$ erreicht ist, beginnt die Zeitmessung (für die Messung der dynamischen Viskosität), die beim Erreichen des Schaltpunktes $s_5$ beendet wird. Während der "Einlauf"-Zeiten zwischen den Schaltpunkten $s_1$ und $s_2$ bzw. $s_3$ und $s_4$ stellt sich die für Messungen (zwischen den Schaltpunkten $s_2$ und $s_3$ bzw. $s_4$ und $s_5$) erwünschte quasi-stationäre Strömung ein. Bei Erreichen des Schaltpunktes $s_5$ schließt das Ventil 18, und das Ventil 24 wird geöffnet, so daß sich der an dem Gasspeicherbehälter 4 anstehende Differenzdruck auf Null abbauen kann, bevor beim Erreichen des Schaltpunktes $s_6$ ein neuer Meßzyklus eingeleitet wird.

Bei der zuvor beschriebenen Messung von Dichte und Viskosität brauchen die als Sensoren dienende Blende 15 und Kapillare 16 nicht so ausgelegt zu werden, daß die Blende selektiv die Dichte und die Kapillare selektiv die Viskosität mißt. Es kann vielmehr ein begrenzter Dichteeinfluß bei der Viskositätsmessung und ein begrenzter Viskositätseinfluß bei der Dichtemessung zugelassen werden. Der sich dadurch ergebende systematische Fehler kann mit Hilfe des Rechners bzw. Prozessors 22 rechnerisch, insbesondere durch Iteration korrigiert werden. Dadurch wird die Auslegung der zugehörigen Sensoren 15 und 16 vereinfacht und die Meßeinrichtung insgesamt kompakter. Die Korrektur der systematischen Fehler kann gerade bei iterativem Verfahren beliebig genau durchgeführt werden, da der Rechner nach Durchführung der Meßzyklen die Iterationsschritte solange fortsetzen, bis die relativen Abweichungen praktisch verschwinden.

Zur Korrektur des systematischen Fehlers bei der Dichtemessung wird in den dem Rechner 22 zugeordneten Speicher eine Kennlinie $\alpha$ (Re) eingegeben, wobei $\alpha$ die Durchflußzahl und Re die Reynoldszahl bedeutet. Die Reynoldszahl ist wie folgt definiert:

$$Re = \frac{\rho \cdot C \cdot L}{\eta} \qquad (4)$$

Hierbei ist C die charakteristische Strömungsgeschwindigkeit und L die charakteristische Blendenabmessung.

Für die Lochblende gilt:

$$C \sim \alpha \sqrt{\frac{\Delta p}{\rho}} \qquad (5)$$

Somit ergibt sich für die Reynoldszahl

$$Re \sim \frac{\alpha \sqrt{\rho} \cdot \sqrt{\Delta p} \cdot L}{\eta} \qquad (6)$$

Bei Eichung mit einem Eichgas (z.B. $CH_4$) in einem Labormeßsystem, wobei sowohl das Druckdifferenzverhältnis als auch das Lochblendenverhältnis 1 sind, erhält man:

$$\frac{Re}{Re_{CH_4}} = \frac{\alpha}{\alpha_{CH_4}} \sqrt{\frac{\rho}{\rho_{CH_4}}} \cdot \frac{\eta_{CH_4}}{\eta} \qquad (7)$$

In einem ersten Iterationsschritt kann mit Hilfe der Meßwerte

$$\rho/\rho_{CH_4}$$

und

$$\eta/\eta_{CH_4}$$

und der ersten Annäherung von

$$\alpha/\alpha_{CH_4} = 1$$

ein erster Näherungswert für

$$Re/Re_{CH_4}$$

ermittelt werden. Bei der bekannten Lochblenden-Kennlinie, die im Speicher gespeichert ist, kann aus

$$\alpha/\alpha_{CH_4} = f(Re/Re_{CH_4})$$

ein neuer Näherungswert für

$$\alpha/\alpha_{CH_4}$$

bestimmt werden. In einem ersten Iterationsschritt (1) wird

$$\rho/\rho_{CH_4}$$

entsprechend der folgenden Beziehung korrigiert:

$$\left(\frac{\rho}{\rho_{CH_4}}\right)_{(1)} = \left(\frac{\alpha}{\alpha_{CH_4}}\right)^2_{(1)} \cdot \left(\frac{\rho}{\rho_{CH_4}}\right)_{(0)} \tag{8}$$

Hierbei sind

$$\rho/\rho_{CH_4\,(0)} = \text{Meßwert}$$

und

$$\rho/\rho_{CH_4\,(1)} = \text{korrigierter Meßwert nach 1.}$$

Iterationsschritt. Mit diesem korrigierten Wert können dann in einem zweiten Interationsschritt die Werte zweiter Näherung für

$$Re/Re_{CH_4},$$

$$\alpha/\alpha_{CH_4}$$

und

$\rho / \rho_{CH_4}$

bestimmt werden. Die Iterationsschritte werden in gleicher Weise zur Eliminierung des systematischen Meßfehlers durch den Durchflußbeiwert $\alpha$ solange rechnerisch fortgesetzt, bis die relative Abweichung von einem (N-1) zum nächstfolgenden Iterationsschritt (N) einen im Rechner bzw. im zugehörigen Speicher gespeicherten Grenzwert unterschreitet. Auf diese Weise braucht die Lochblende nicht mit exakt konstantem Durchflußbeiwert $\alpha$ über einen weiten Reynoldszahlbereich ausgelegt zu werden, sondern es kann ein begrenzter Viskositätseinfluß zugelassen werden, dessen systematischer Fehler durch die zuvor beschriebene Verfahrensweise iterativ korrigiert wird.

Mit Hilfe der Meßgrößen $\rho$ und $\eta$ kann auch der $\rho$ -Einfluß auf die Kapillarenströmung durch Iteration rechnerisch korrigiert werden.

Die Strömung durch Kapillaren wird nur dann ausreichend genau nach dem Hagen-Poiseuille-Gesetz beschrieben, wenn weitere Einflüsse, insbesondere diejenigen der Dichte, vergleichsweise gering sind. Solche Einflüsse entstehen bei den Strömungsvorgängen im Einlauf und Auslauf der Kapillaren. Im Einlauf ist einerseits eine Druckdifferenz zur Beschleunigung der Strömung auf die Geschwindigkeit im engen Querschnitt der Kapillaren und andererseits eine etwas größere Druckdifferenz zur Ausbildung des parabelförmigen laminaren Strömungsprofiles erforderlich. Im Auslauf der Kapillaren werden diese Druckdifferenzen in der Regel nicht kompensiert. Diese Druckdifferenzen werden neben den vorgegebenen geometrischen Verhältnissen nur noch von der Gasdichte beeinflußt. Nach der Literatur gilt für die Dichte-abhängige Einlaufdruckdifferenz:

$$\Delta p_\rho = (1 + 1{,}15)\,\frac{\rho}{2}\cdot\left(\frac{\dot V}{A}\right)^2 \qquad (9)$$

nach Hagen-Poiseuille gilt für die viskositätsabhängige Druckdifferenz in der Kapillare mit kreisrundem Querschnitt:

$$\Delta p_\eta = 32 \cdot (\ell \cdot \eta / d^2)(\dot V / A) \qquad (10)$$

Damit für den gesamten Druckabfall in der Kapillaren

$$\Delta p = \Delta p_\rho + \Delta p_\eta \qquad (11)$$

die für die Viskositätsmessung gewünschte Proportionalität $\eta \sim \Delta p / \dot V$ gilt, muß $\Delta p_\rho << \Delta p_\eta$ sein. Diese Bedingung ist erfüllt für

$$\frac{\Delta p_\rho}{\Delta p_\eta} = \frac{2{,}15 \cdot \frac{\rho}{2} \cdot \left(\frac{\dot V}{A}\right)^2}{32 \cdot \frac{\ell \cdot \eta}{d^2} \cdot \frac{\dot V}{A}} << 1 \qquad (12)$$

Aus (10) ergibt sich:

$$\frac{\dot V}{A} = \frac{\Delta p_\eta}{32 \cdot \frac{\ell \cdot \eta}{d^2}} \qquad (13)$$

Zur iterativen Korrektur des Dichteeinflusses auf die Viskositätsmessung geht man von folgenden Startwerten aus:

$\rho$ , gewonnen aus der Dichtemessung des vorangegangenen Meßzyklus

$\eta^{(o)}$ aus dem vorengegangenen Meßzyklus ohne rechnerische Korrektur des Dichteeinflusses

$\Delta p_\eta^{(o)} = \Delta p$ = mittlere Druckdifferenz während des -Meßzyklus (Kapselfeder-Kenngröße)

mit der Zahl der Iterationsschritte $N \geq 1$ wird in jedem Iterationsschritt wie folgt vorgegangen:

1) $$\Delta p_\rho^{(N)} = 2{,}15 \cdot \frac{\rho}{2}\left(\frac{\Delta p_\eta^{(N-1)}}{32\,\frac{\ell}{d^2}\cdot \eta^{(N-1)}}\right)^2$$

2) $$\Delta p_\eta^{(N)} = \Delta p - \Delta p_\rho^{(N)}$$

$$3) \qquad \eta^{(N)} = \eta^{(N-1)} \cdot \frac{\Delta p_\eta^{(N)}}{\Delta p_\eta^{(N-1)}}$$

Diese Iteration wird solange durchgeführt, bis die relative Abweichung in $\eta$

$$\frac{\eta^{(N)} - \eta^{(N-1)}}{\eta^{(N)}} = \varepsilon_\eta$$

einen vorgegebenen Genauigkeitsgrenzwert $\varepsilon g$ unterschreitet.

Wie oben gesagt, werden sowohl die Meßwerte der Dichte und Viskosität als auch die für die Iteration benötigten Startwerte und Iterationsergebnisse in einem in der Zeichnung nicht gesondert dargestellten, jedoch dem Rechner 22 zugeordneten Speicher gespeichert.

Das oben erwähnte Kalibrieren kann in der Praxis dadurch erfolgen, daß man für ein Prüfgas bekannter Gaseigenschaften die Meßvorrichtung derart einstellt, daß sich bestimmte Sollzeiten in den Zeitmeßphasen zwischen den o.g. Schaltpunkten $s_2$ und $s_3$ bzw. $s_4$ und $s_5$ ergeben. In den nachfolgenden Meßzyklen sind die zwischen den Schaltpunkten ermittelten Istzeiten ein Maß für die gesuchten Gaseigenschaften.

Alle Ventile, insbesondere aber die Ventile 17 und 18 sollten schnell-schließend und öffnend ausgebildet sein, um Totzeiten zu vermeiden bzw. zu minimieren. Die Ventile bzw. deren Antriebe sollten ohne Eigenerwärmung arbeiten und extrem dicht schließen, um Meßwertverfälschungen zu vermeiden. Alle Ventile sind in vibrationsarmer Ausführung vorgesehen, um eine Beeinflussung der Kapselfederkennlinie zu vermeiden. Die Werkstoffe der mit dem zu untersuchenden Gas in Berührung kommenden Komponenten der Meßvorrichtung gemäß Figur 1 sind so gewählt, daß sie die in Betracht kommenden Meßgase nicht absorbieren.

Die erfindungsgemäß für die Messung von Gaseigenschaften vorgesehene Zeiterfassung zeichnet sich durch eine im Vergleich zu anderen Meßgrößen hohe Auflösung aus.

## Patentansprüche

1. Verfahren zum Messen wenigstens einer Gaseigenschaft, beispielsweise der Dichte von Gasen, unter Verwendung eines Gasspeicherbehälters, wobei

der Gasspeicherbehälter mit dem zu untersuchenden Gas gefüllt,

nach dem Füllen des Gasspeicherbehälters die Gaszufuhr unterbrochen,

das Gas sodann über mindestens einen Strömungswiderstand abströmen gelassen wird, wobei der Gasspeicherbehälter eine Volumenverringerung erfährt,

die Zeit $\Delta t$ für eine Volumenverringerung gemessen und die gesuchte Gaseigenschaft aus der gemessenen Zeit $\Delta t$ und der Volumenverringerung bestimmt wird,

**dadurch gekennzeichnet,**

daß ein Gasspeicherbehälter verwendet wird, der in vorgegebener Abhängigkeit von Druckdifferenzen ($\Delta p$) zwischen seinem Innenraum und seiner Umgebungsatmosphäre Volumenänderungen ($\Delta V$) erfährt,

daß die genannte vorgegebene Abhängigkeit ($\Delta p/\Delta V$) des Gasspeicherbehälters bestimmt wird,

daß danach der Meßzyklus unter Füllen des Gasspeicherbehälters mit dem zu untersuchenden Gas begonnen wird, wobei das Volumen des Gasspeicherbehälters und die Druckdifferenz nach der zuvor bestimmten Abhängigkeit vergrößert werden,

daß während des Abströmens des Gases sowohl das Volumen des Gasspeicherbehälters als auch die Druckdifferenz nach der vorgegebenen Abhängigkeit verringert und die Zeit $\Delta t$ für eine vorgegebene Volumenverringerung oder eine vorgegebene Druckdifferenzverringerung gemessen wird und

daß der Volumenstrom und die gesuchte Gaseigenschaft aus der gemessenen Zeit $\Delta t$ und wahlweise der genannten Volumenverringerung oder der zugehörigen Druckdifferenzverringerung bestimmt werden.

2. Verfahren nach Anspruch 1, dadurch gekennzeichnet, daß als Gasspeicherbehälter eine Kapselfeder verwendet wird.

3. Verfahren nach Anspruch 1 oder 2, dadurch gekennzeichnet, daß die Grenzen der wenigstens einen Zeitmessung in einen Bereich gelegt werden, in welchem sich die Druckdifferenz im wesentlichen linear mit der Zeit ändert.

4. Verfahren nach einem der Ansprüche 1 bis 3, dadurch gekennzeichnet, daß das zu untersuchende Gas aus dem Gasspei cherbehälter nacheinander durch zwei unterschiedliche Strömungswiderstände geleitet wird.

5. Verfahren nach Anspruch 4, dadurch gekennzeichnet, daß das zu untersuchende Gas aus dem Gasspeicherbehälter zunächst durch einen Turbulenzwiderstand und danach durch einen Laminarwiderstand geleitet wird.

6. Verfahren nach einem der Ansprüche 1 bis 5, dadurch gekennzeichnet, daß die Gaszufuhr zum Gasspeicherbehälter in Abhängigkeit von einem vorgegebenen Fülldruck im Gasspeicherbehälter unterbrochen

wird.

7. Verfahren nach einem der Ansprüche 1 bis 6, dadurch gekennzeichnet, daß während des Füllvorganges des Gasspeicherbehälters gleichzeitig auch der mindestens eine Strömungswiderstand mit dem Füllgas beaufschlagt wird.

8. Verfahren nach einem der Ansprüche 1 bis 7, dadurch gekennzeichnet, daß zur Begrenzung von Kompressibilitätseinflüssen der Fülldruck im Gasspeicherbehälter auf maximal ca. 100 mbar begrenzt wird.

9. Verfahren nach einem der Ansprüche 1 bis 8, dadurch gekennzeichnet, daß die Zeitmessung nach Erreichen einer quasi stationären Strömung durch den mindestens einen Strömungswiderstand beginnt.

10. Verfahren nach einem der Ansprüche 1 bis 9, dadurch gekennzeichnet, daß zu untersuchendes Gas aus dem Gasspeicherbehälter in zeitlich versetzten Meßphasen einem Dichtesensor (Blende) und einem Viskositätssensor (Kapillare) zugeführt wird und für die Dichte und die Viskosität repräsentative Meßwerte aufgenommen werden, wobei ein begrenzter Viskositätseinfluß auf den Dichtesensor und ein begrenzter Dichteeinfluß auf den Viskositätssensor zugelassen werden, und daß die aus diesen Einflüssen resultierenden Meßfehler der aufgenommenen Meßwerte rechnerisch korrigiert werden.

11. Verfahren nach Anspruch 10, dadurch gekennzeichnet, daß jeder Meßwert zunächst als näherungsweiser Startwert für einen ersten Iterationsschritt benutzt und aus dem Iterationsergebnis ein korrigierter Startwert für einen zweiten Iterationsschritt abgeleitet wird und daß die Iteration solange fortgesetzt wird, bis die relative Abweichung zwischen aufeinanderfolgenden Iterationsschritten (N und N-1) kleiner als ein vorgegebener Genauigkeitsgrenzwert ist.

12. Verfahren nach Anspruch 11, gekennzeichnet, daß man die Dichte- und Viskositätsmessugen zunächst an einem Eichgas (z. B. $CH_4$) mit bekannten physikalischen Eigenschaften durchführt, die daraus gewonnenen Werte als Bezugswerte speichert und die Dichte- und Viskositätsmessungen in derselben Anordnung an dem zu untersuchenden Gas wiederholt und ebenfalls speichert, daß man, ausgehend von einem ersten Näherungswert für das Durchflußzahlverhältnis

$$\alpha / \alpha_{CH_4}$$

unter Verwendung einer gespeicherten Dichtesensor-Kennlinie sowie der Meßwertverhältnisse

$$\rho / \rho_{CH_4}$$

und

$$\eta / \eta_{CH_4}$$

eine iterative Korrektur solange durchführt, bis die relative Abweichung den vorgegebenen Genauigkeitsgrenzwert unterschritten hat.

13. Verfahren nach Anspruch 11 oder 12, dadurch gekennzeichnet, daß man die Iteration mit den folgenden Startwerten beginnt: $\rho$ und $\eta$ aus den vorangegangenen Meßzyklen mit $\eta$ ohne Korrektur des Dichteeinflusses und einer viskositätsabhängigen Druckdifferenz $\Delta p_\eta^{(o)}$ gleich einer als apparative Kenngröße vorausbestimmten mittleren Druckdifferenz $\Delta p$, daß man danach eine dichteabhängige erste Einlaufdruckdifferenz $\Delta p_\rho$ nach der Gleichung

$$\Delta p_\rho^{(N)} = 2{,}15 \cdot \frac{\rho}{2}\left(\frac{\Delta p_\eta^{(N-1)}}{32 \frac{l}{d^2} \cdot \eta^{(N-1)}}\right)^2$$

berechnet, wobei N die Zahl der Iterationsschritte, 1 die Länge der Kapillare, d der Kapillarendurchmesser ist, und eine viskositätsabhängige Druckdifferenz in der Kapillare nach der Gleichung

$$\Delta p_\eta^{(N)} = \Delta p - \Delta p_\rho^{(N)}$$

berechnet und daß man über

$$\eta^{(N)} = \eta^{(N-1)} \frac{\Delta p_\eta^{(N)}}{\Delta p_\eta^{(N-1)}}$$

die relative Abweichung

$$\frac{\eta^{(N)} - \eta^{(N-1)}}{\eta^{(N)}}$$

bestimmt und mit dem Genauigkeitsgrenzwert vergleicht.

14. Vorrichtung zum Messen von Gaseigenschaften, die aus einem Volumenstrom und/oder einer Druckdifferenz bestimmbar sind mit einer einen Gasspeicherbehälter (4) enthaltenden Dosiervorrichtung, wenigstens einem Strömungswiderstand (15, 16), der an den Gasspeicherbehälter (4) angebunden ist, und einer Zeitmeßvorrichtung (11), die in Abhängigkeit von vorgegebenen Volumina des Gasspeicherbehälters (4) ein- und ausschaltbar ist,

daß ein absperrbarer Gaseinlaß (2) vorgesehen ist, über den das Volumen des Gasspeicherbehälters (4) in Abhängigkeit von Druckdifferenzen zwichen dem Speicherbehälter-Innenraum (7) und der Umgebungsatmosphäre füllbar ist;

**dadurch gekennzeichnet**, daß der Gasspeicherbehälter (4) so ausgebildet ist, daß sein Innenraum (7) in vorgegebener Abhängigkeit von Druckdifferenzen (Δp) zur Umgebungsatmosphäre Volumenänderungen (ΔV) erfährt,

daß die Zeitmeßvorrichtung (11) wahlweise in Abhängigkeit von vorgegebenen Volumina des Gasspeicherbehälters (4) oder von Druckdifferenzen ein- und ausschaltbar ist; und

daß Mittel zur Bestimmung einer Abhängigkeit von Druckdifferenzen (Δp) zwischen dem Gasspeicherbehälter-Innenraum (7) und der Umgebungsatmosphäre zu dabei auftretenden Volumenänderungen (ΔV) des Gasspeicherbehälters (4) vorgesehen sind.

15. Vorrichtung nach Anspruch 14, dadurch gekennzeichnet, daß der Gasspeicherbehälter (4) als Kapselfeder ausgebildet ist, deren aus lenkbarer Membran ein Wegaufnehmer (8) zugeordnet ist.

16. Vorrichtung nach Anspruch 15, dadurch gekennzeichnet, daß die Zeitmeßvorrichtung (11) in Abhängigkeit von vorgegebenen Stellungen des Wegaufnehmers (B) betätigbar ist.

17. Vorrichtung nach einem der Ansprüche 14 bis 16, dadurch gekennzeichnet, daß ein Strömungswiderstand als Laminarwiderstand (16) ausgebildet ist.

18. Vorrichtung nach einem der Ansprüche 14 bis 17, dadurch gekennzeichnet, daß ein Strömungswiderstand als Turbulenzwiderstand (15) ausgebildet ist.

19. Vorrichtung nach den Ansprüchen 17 und 18, dadurch gekennzeichnet, daß ein Laminarwiderstand (16) und ein Turbulenzwiderstand (15) parallel zueinander an dem Gasspeicherbehälter (4) angebunden und mittels ihnen jeweils zugeordneter Absperrventile (18, 17) von einem Steuergerät (22, 23) nacheinander in den Strömungsweg des aus dem Gasspeicherbehälter abströmenden Gases einschaltbar sind.

20. Vorrichtung nach einem der Ansprüche 14 bis 19, dadurch gekennzeichnet, daß ein den Umgebungsdruck des Gasspeicherbehälters (4) erfassender Druckaufnehmer (21) vorgesehen ist.

21. Vorrichtung nach einem der Ansprüche 14 bis 20, dadurch gekennzeichnet, daß der Gasspeicherbehälter (4) in einem thermostatisierten Gehäuse (19) angeordnet ist.

22. Vorrichtung nach Anspruch 21, dadurch gekennzeichnet, daß im Gehäuse (19) ein Temperaturfühler (20) angeordnet ist, der mit einem Temperaturregler und/oder einem Rechengerät (23) gekoppelt ist.

23. Vorrichtung nach einem der Ansprüche 14 bis 22, dadurch gekennzeichnet, daß ein Rechengerät (23) zur Meßwertverarbeitung mit den Meßwertaufnehmern (8, 20, 21, 26) gekoppelt ist.

24. Vorrichtung nach Anspruch 23, dadurch gekennzeichnet, daß eine Steuereinheit (22) zur Betätigung der den Absperrorganen (1, 17, 18, 24) zugeordneten Stellglieder mit dem Rechengerät (23) gekoppelt ist.

25. Vorrichtung nach einem der Ansprüche 14 bis 24, dadurch gekennzeichnet, daß eine absperrbare Spülleitung an das Leitungssystem (5, 6, 12) zwischen dem Gasspeicherbehälter (4) und dem mindestens einen Strömungswiderstand (15, 16) angebunden ist.

## Claims

1. A method for measuring at least one gas property, such as the density of a gas, using a gas holding chamber, where

the gas holding chamber is filled with the gas to be measured,

gas supply is interrupted after filling said gas holding chamber,

the gas is thereupon permitted to discharge across at least one flow resistor, the volume of the gas holding chamber thereby being decreased,

the time Δt taken for the volume of said chamber to decrease is measured and the desired gas property is determined from the measured time Δt and the decrease in volume

**characterized** in that

a gas holding chamber is used undergoing changes in volume (ΔV) which are in a given relation to press-

ure differences ($\Delta p$) between its interior and the ambient atmosphere,

said given relation ($\Delta p/\Delta V$) of the gas holding chamber is determined,

the measurement cycle implying filling the gas holding chamber with the gas to be measured is thereafter commenced, the volume of the gas holding chamber and said pressure difference being increased in accordance with the relation determined as aforesaid,

both the volume of the gas holding chamber and the pressure difference are decreased in accordance with the given relation as the gas is discharged and the time $\Delta t$ for a predetermined decrease in volume or a determined decrease in the pressure difference is measured,

the volume rate of flow and the desired gas property are determined from the measured time $\Delta t$ and selectively from the said decrease in volume or said associated decrease in pressure difference.

2. A method according to claim 1 characterized in that said gas holding chamber is an aneroid capsule.

3. A method according to claim 1 or 2 characterized in that the commencement and the end of said at least one time measurement are within a period in which the change in the pressure difference is a substantially linear function of time.

4. A method according to any one of claims 1 through 3 characterized in that the gas to be measured is discharged from the gas holding chamber across two different successive flow restrictors.

5. A method according to claim 4 characterized in that the gas to be measured is discharged from the gas holding chamber first across a turbulent flow restrictor and thereafter across a laminar flow restrictor.

6. A method according to any one of claims 1 through 5 characterized in that gas supply to the gas holding chamber is interrupted as a function of a predetermined filling pressure inside the gas holding chamber.

7. A method according to any one of claims 1 through 6 characterized in that the at least one flow restrictor is exposed to gas from the gas holding chamber as the gas holding chamber is being filled.

8. A method according to any one of claims 1 through 7 characterized in that the pressure inside the gas holding chamber is limited to a maximum pressure of approximately 100 mbar to thereby limit the effect of compressibility.

9. A method according to any one of claims 1 through 8 characterized in that the measurement of time commences upon flow across said at least one flow restrictor having become quasi-stationary.

10. A method according to any one of claims 1 through 9 characterized in that the gas to be measured is directed from the gas holding chamber during different measurement intervals to a density sensing device (diaphragm) in a first measurement phase and to a viscosity sensing device (capillary tube) and values representative of density and of viscosity are measured; a limited influence of viscosity on the density sensing device and a limited influence of density on the viscosity sensing device being tolerated and the errors of the values so measured resulting from said influences are corrected for by computational means.

11. A method according to claim 10 characterized in that each value so measured is used as a first approximation for a first iterative step and the improved approximation resulting from said first iterative step is used as corrected approximation for a second iterative step and the iterations are continued until the relative difference between the values obtained by two successive iterative steps (n and n - 1) is smaller than a predetermined maximum difference.

12. A method according to claim 11 characterized in that the density and the viscosity of a calibration gas of known physical properties such as $CH_4$ are first measured, the values obtained through said measurements are stored as reference values and the density and viscosity of the gas to be measured are then measured using the same arrangement and also stored and, starting from a first approximation of the flow coefficient ratio

$$\alpha / \alpha_{CH_4}$$

using a stored characteristic curve for the density sensing device and the measured value ratios

$$\rho / \rho_{CH_4}$$

and

$$\eta / \eta_{CH_4},$$

an iterative correction is made until the relative difference between two successive iterative steps is less than a predetermined maximum difference.

13. A method according to claim 11 or 12 characterized in that the initial values for the iteration are $\rho$ and $\eta$ as previously measured, the initial value of $\eta$ not being corrected for the influence of density, and predetermined viscosity-dependent pressure difference $\Delta p \eta^{(o)}$ equal to an average pressure difference previously determined as a constant of the apparatus used for the application of the method claimed herein, a density-dependent first inlet pressure difference is thereupon calculated, using the equation

$$\Delta p_{\rho}^{(N)} = 2.15 \cdot \frac{\rho}{2} \left[ \frac{\Delta p_{\eta}^{(N-1)}}{32 \frac{L}{d^2} \cdot \eta^{(N-1)}} \right]^2$$

N being the number of iterations, L being the length of the capillary tube, d being the diameter of said capillary tube, a viscosity-dependent pressure difference in the capillary tube is thereupon calculated using the equation

$\Delta p_{\eta}^{(N)} = \Delta p - \Delta p_{\rho}^{(N)}$ the relative difference

$$\eta^{(N)} = \eta^{(N-1)} \cdot \frac{\Delta p_{\eta}^{(N)}}{\Delta p_{\eta}^{(N-1)}}$$

is thereafter calculated with

$$\frac{\eta^{(N)} - \eta^{(N-1)}}{\eta^{(N)}}$$

and said relative difference is compared with the predetermined maximum difference.

14. An apparatus for measuring gas properties determinable from a volume rate of flow and/or a pressure difference, comprising a dosing device containing a gas holding chamber (4), at least one flow restrictor (15, 16) connected with said gas holding chamber (4), a time measuring device (11) which may be switched on and off as a function of predetermined volumes of the gas holding chamber (4) and a closable gas inlet (2) across which the gas holding chamber (4) is fillable as a function of pressure differences between the interior (7) of the gas holding chamber and the ambient atmosphere

**characterized in that**

the gas holding chamber (4) is provided such that its interior (7) undergoes changes in volume ($\Delta V$) as a given function of pressure differences ($\Delta p$) relative to the ambient atmosphere,

the time measuring device (11) may be switched on and off selectively as a function of predetermined volumes of the gas holding chamber (4) or pressure differences and

means are provided for determining a correlation between pressure differences ($\Delta p$) between the pressure in the inner chamber gas holding (7) of the gas holding chamber and ambient atmosphere and the associated changes in the volume ($\Delta V$) of the gas holding chamber.

15. An apparatus according to claim 14 characterized in that the gas holding chamber (4) is an aneroid capsule, the movable diaphragm of said capsule being associated with a displacement measurement device (8).

16. An apparatus according to claim 15 characterized in that the time measuring device (11) may be actuated as a function of predetermined positions of said displacement measurement device (8).

17. An apparatus according to any one of claims 14 through 16 characterized in that a flow restrictor is a laminar flow restrictor (16)

18. An apparatus according to any one of the claims 14 through 17 characterized in that a flow restrictor is a turbulent flow restrictor (15).

19. An apparatus according to claims 17 and 18 characterized in that the laminar flow restrictor (16) and the turbulent flow restrictor (15) are arranged in parallel and connected with the gas holding chamber (4) and may be integrated successively in the gasway carrying the gas being discharged from said gas holding chamber by means of isolating valves (18,17) controlled by control devices (22,23), each such isolating valve being associated with one of said flow restrictors.

20. An apparatus according to any one of claims 14 through 19 characterized in that a pressure pick-up (21) to measure the ambient pressure of the gas holding chamber (4) is provided.

21. An apparatus according to any one of claims 14 through 20 characterized in that the gas holding chamber (4) is arranged inside a thermostated casing (19).

22. An apparatus according to claim 21 characterized in that a temperature sensor (20) is arranged inside the casing (19) and coupled with a temperature controller and/or a computing device (23).

23. An apparatus according to any one of claims 14 through 22 characterized in that a computing device (23) is coupled with pick-up devices (8, 20, 21, 26) for processing the values measured thereby.

24. An apparatus according to claim 23 characterized in that a control unit (22) provided to control the actuators associated with the isolating means (1, 17, 18, 24) is coupled with the computing device (23).

25. An apparatus according to any one of claims 14 through 24 characterized in that a closable purge line is connected with the lines (5, 6, 12) between the gas holding chamber (4) and the at least one flow restrictor (15, 16).

## Revendications

1. Procédé pour la mesure d'au moins une propriété de gaz, telle que la densité, à l'aide d'un réservoir de gaz, ce réservoir de gaz étant rempli du gaz à mesurer,

l'amenée de gaz étant interrompue après le remplissage,

le gaz étant alors laissé sortir à travers au moins une résistance d'écoulement, le volume du réservoir se diminuant,

le temps $\Delta t$ de cette diminution étant mesuré et la propriété à mesurer déterminée à partir du temps $\Delta t$ mesuré et de la diminution du volume,

**caractérisé par le fait**

qu'un réservoir est utilisé qui en fonction préétablie de la différence de pression ($\Delta p$) entre son espace intérieure et l'atmosphère ambiante subit des modifications volumiques ($\Delta V$), que cette fonction préetablie ($\Delta p/\Delta V$) du réservoir est déterminée,

qu'après cette détermination le cycle de mesure commence par le remplissage du réservoir avec le gaz à mesurer, le volume du réservoir et la différence de pressions étant alors augmentés selon la fonction préétablie,

que pendant la sortie du gaz tant le volume du réservoir que la différence de pression sont diminués suivant la fonction préétablie et le temps $\Delta t$ requis pour que cette diminution prédéterminée du volume ou de la différence de pression se produise est mesuré,

et que le débit volumique et la propriété du gaz recherchée sont déterminés à partir du temps $\Delta t$ mesuré et, sélectivement de la diminution du volume ou de la diminution de la différence de pression s'y rapportant.

2. Procédé selon la revendication 1, caractérisé par le fait que le réservoir est une capsule anéroide

3. Procédé selon l'une quelconque des revendications 1 ou 2, caractérisé par le fait que le commencement et la fin d'au moins une mesure de temps se situent à l'intérieur d'une période pendant laquelle la modification de la différence de pression est essentiellement une fonction linéaire du temps.

4. Procédé selon l'une quelconque des revendications 1 à 3, caractérisé par le fait que le gaz à mesurer provenant du réservoir passe successivement par deux résistances d'écoulement différentes.

5. Procédé selon la revendication 4, caractérisé par le fait que le gaz à mesurer provenant du réservoir passe d'abord par une résistance d'écoulement turbulente et ensuite par une résistance d'écoulement laminaire.

6. Procédé selon l'une quelconque des revendications 1 à 5, caractérisé par le fait que l'amenée du gaz au réservoir est interrompue en fonction d'une pressure de remplissage prédéterminée à l'intérieur du réservoir.

7. Procédé selon l'une quelconque des revendications 1 à 6, caractérisé par le fait que pendant le remplissage du réservoir au moins une résistance d'écoulement est simultanément exposée au gaz du réservoir.

8. Procédé selon l'une quelconque des revendications 1 à 7, caractérisé par le fait que la pression de remplissage à l'intérieur du réservoir est limitée à environ 100 mbar au maximum pour limiter l'effet de compressibilité.

9. Procédé selon l'une quelconque des revendications 1 à 8, caractérisé par le fait que la mesure du temps commence lorsque l'écoulement du gaz à travers la résistance ou les résistances d'écoulement est devenu quasi-stationnaire.

10. Procédé selon l'une quelconque des revendications 1 à 9, caractérisé par le fait que le gaz à mesurer provenant du réservoir passe pendant différents intervalles de mesure décalés dans le temps par un capteur de masse volumique (diaphragme) et un capteur de viscosité (tube capillaire) et que des valeurs représentatives de la masse volumique et de la viscosité sont mesurées, étant donné qu'une influence limitée de la viscosité sur le capteur de masse volumique et une influence limitée de la masse volumique sur le capteur de la viscosité sont tolérées et que les erreurs de mesure résultant de ces influences sont corrigées par le calcul.

11. Procédé selon la revendication 10, caractérisé par le fait que chaque valeur ainsi mesurée est utilisée

en tant que première approximation d'une première itération et qu'une valeur approximative corrigée servant de base à une deuxième itération est dégagée du résultat de la première itération et que l'itération est poursuivie jusqu'à ce que la différence relative entre itérations successives (n et n-1) est plus petite qu'une différence maximale prédéterminée.

12. Procédé selon la revendication 11, caractérisé par le fait que les mesures de la masse volumique et de la viscosité sont d'abord effectuées avec un gaz de calibrage tel que le $CH_4$ dont les propriétés physiques sont connues, que les valeurs ainsi mesurées sont mémorisées comme valeurs de référence et que les mesures de la masse volumique et la viscosité sont répétées dans les mêmes conditions avec le gaz à mesurer et également mémorisées, qu'à partir d'une première approximation du rapport entre les coefficients de débit

$$\alpha / \alpha_{CH_4}$$

en utilisant une courbe caractéristique du capteur de masse volumique également mémorisée et le rapport des valeurs mesurées

$$\rho / \rho_{CH_4}$$

et

$$\eta / \eta_{CH_4}$$

il est procédé à une correction itérative jusqu'à ce que la différence relative entre deux itérations successives est plus petite qu'une différence maximale prédéterminée.

13. Procédé selon l'une quelconque des revendications 11 ou 12, caractérisé par le fait que les valeurs ρ et η provenant des cycles de mesure précédants, avec η comme précédemment mesuré mais sans correction de l'influence de la masse volumique et une différence de pression $\Delta p\eta^{(o)}$, cette différence étant fonction de la viscosité, égale à une différence de pression Δp moyenne précédemment déterminée et considérée comme étant une constante de l'appareillage employé pour le procédé revendiqué, sont utilisées pour une approximation par itération qu'une première différence de pression d'entrée $\Delta p\rho$, fonction de la masse volumique, est ensuite déterminée par calcul selon l'équation

$$\Delta p_{\rho}^{(N)} = 2.15 \cdot \frac{\rho}{2} \left[ \frac{\Delta p_{\eta}^{(N-1)}}{32 \frac{L}{d^2} \cdot \eta^{(N-1)}} \right]^2$$

dans laquelle N est le nombre d'itérations, L la longeur du tube capillaire et d le diamètre du tube capillaire, qu'une différence de pression pour le tube capillaire, fonction de la viscosité, étant ensuite déterminée par calcul selon l'équation

$$\Delta p_{\eta}^{(N)} = \Delta p - \Delta p_{\rho}^{(N)}$$

et en calculant l'équation

$$\eta^{(N)} = \eta^{(N-1)} \cdot \frac{\Delta p_{\eta}^{(N)}}{\Delta p_{\eta}^{(N-1)}}$$

la différence relative

$$\frac{\eta^{(N)} - \eta^{(N-1)}}{\eta^{(N)}}$$

est ensuite calculée et comparée avec une différence relative maximale prédéterminée.

14. Dispositif pour la mesure de propriétés de gaz qui peuvent être déterminés à partir d'un débit volumique et/ou d'une différence de pression, comprenant un appareil de dosage avec un réservoir à gaz (4), au moins une résistance d'écoulement (15, 16) connectée avec ledit réservoir à gaz (4), et une horloge (11) pouvant être enclenchée et déclenchée en fonction des volumes prédéterminées dudit réservoir (4) et une entrée du gaz (2) pouvant être fermée, par l'intermédiaire de laquelle le volume dudit réservoir (4) peut être rempli en fonction des différences de pression entre l'espace intérieure (7) dudit réservoir et l'atmosphère ambiante,

**caractérisé par le fait**

que le réservoir à gaz (4) est congru de manière que son espace intérieure (7) subit des changements de volume ($\Delta V$) en fonction prédéterminée de différences de pression ($\Delta p$) par rapport à l'atmosphère ambiante,

que l'horloge (11) peut être enclenchée ou déclenchée en fonction prédéterminée de volumes dudit réservoir (4) ou de différences de pression et

que des moyens sont prévus pour déterminer la correlation entre les différences de pression ($\Delta p$) entre l'espace intérieure (7) dudit réservoir à gaz et l'atmosphère ambiante aussi que les modifications de volume ($\Delta V$) dudit réservoir à gaz (4) s'y rapportant.

15. Dispositif selon la revendication 14 caractérisé par le fait que le réservoir à gaz (4) est une capsule anéroide dont le diaphragme mobile est couplé avec un capteur de déplacement (8).

16. Dispositif selon la revendication 15 caractérisé par le fait que l'horloge (11) peut être actionnée en fonction des positions prédéterminées du capteur de déplacement (8).

17. Dispositif selon l'une quelconque des revendications 14 à 16 caractérisé par le fait que l'une des résistances d'écoulement est une résistance laminaire (16).

18. Dispositif selon l'une quelconque des revendications 14 à 17 caractérisé par le fait que l'une des résistances d'écoulement est une résistance turbulente (15).

19. Dispositif selon les revendications 17 et 18 caractérisé parle fait qu'une résistance laminaire (16) et une résistance turbulente (15) sont disposées en parallèle et connectées avec ledit réservoir à gaz (4) et peuvent être successivement intégrées dans le courant de gaz sortant dudit réservoir à l'aide de robinets d'arrêt (18, 17) qui leur sont associés et actionnés par un appareillage de commande (22, 23).

20. Dispositif selon l'une quelconque des revendications 14 à 19 caractérisé par le fait qu'un capteur de pression (21) est prévu pour mesurer la pression ambiante à laquelle ledit réservoir (4) est exposé.

21. Dispositif selon l'une quelconque des revendications 14 à 20 caractérisé par le fait que ledit réservoir (4) est disposé à l'intérieur d'un carter thermostaté (19).

22. Dispositif selon la revendication 21 caractérisé par le fait que le carter (19) est muni d'un capteur de température (20) couplé avec un régulateur de la température et/ou un moyen calculateur (23).

23. Dispositif selon l'une quelconques des revendications 14 à 22 caractérisé par le fait qu'un moyen calculateur est couplé avec les capteurs (8, 20, 21, 26) pour traiter les valeurs mesurées.

24. Dispositif selon la revendication 23 caractérisé par le fait qu'une unité de commande (22) pour actionner les opérateurs associés aux robinets (1, 17, 18 24) est couplée avec le moyen calculateur (23).

25. Dispositif selon l'une quelconque des revendications 14 à 24 caractérisé par le fait qu'une conduite de purge pouvant être fermée est connectée avec les conduites (5, 6, 12) entre ledit réservoir (4) et la résistance ou les résistances d'écoulement (15, 16).

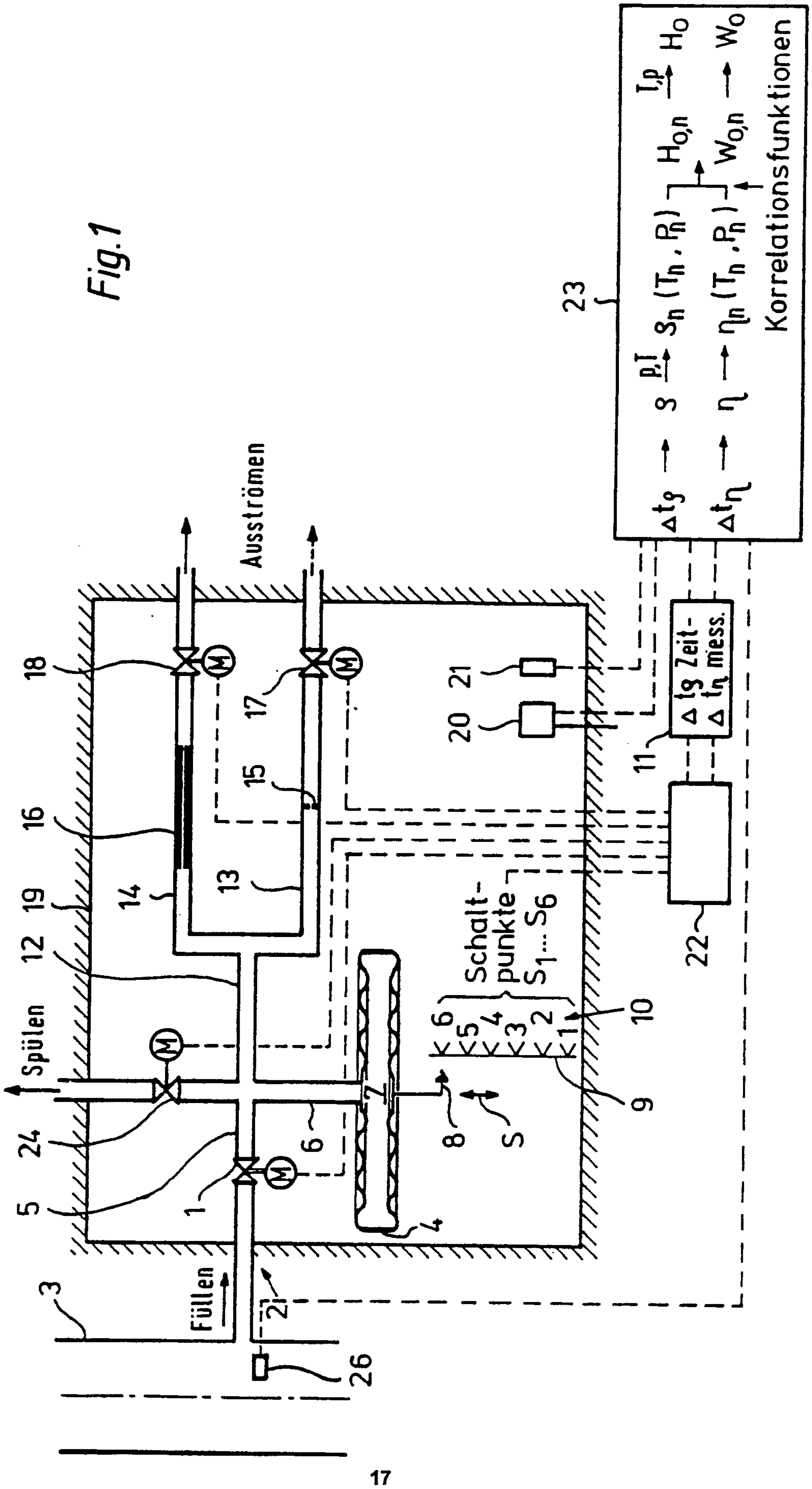

Ausströmen
Spülen
Füllen
Schalt-
punkte
$S_1 \ldots S_6$
$\Delta t_\varrho$ Zeit-
$\Delta t_\eta$ mess.
Korrelationsfunktionen


Fig.1

Fig. 2

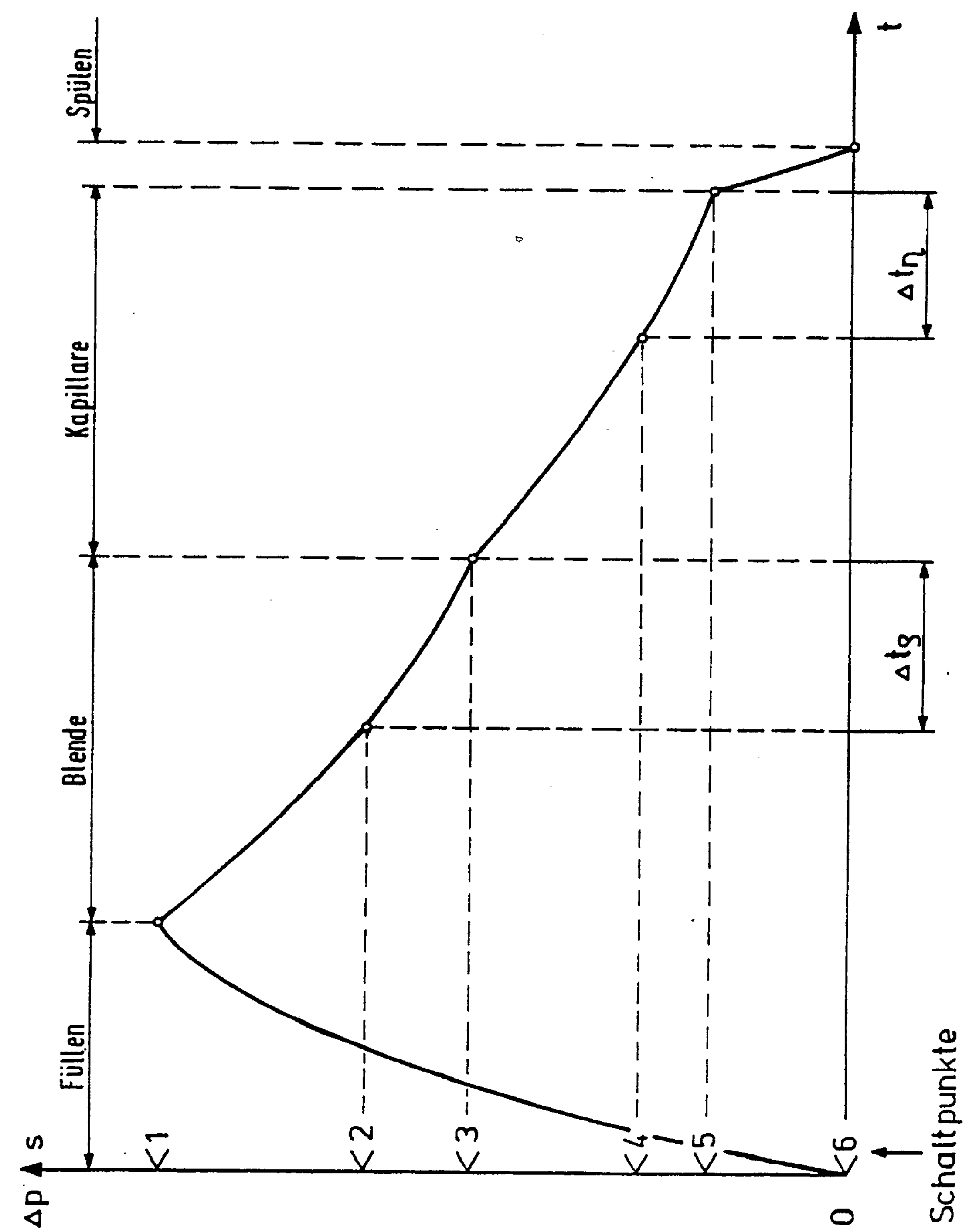
Δp
s
0
1
2
3
4
5
6
Schaltpunkte
Füllen
Blende
Kapillare
Spülen
t
Δtg
Δtη

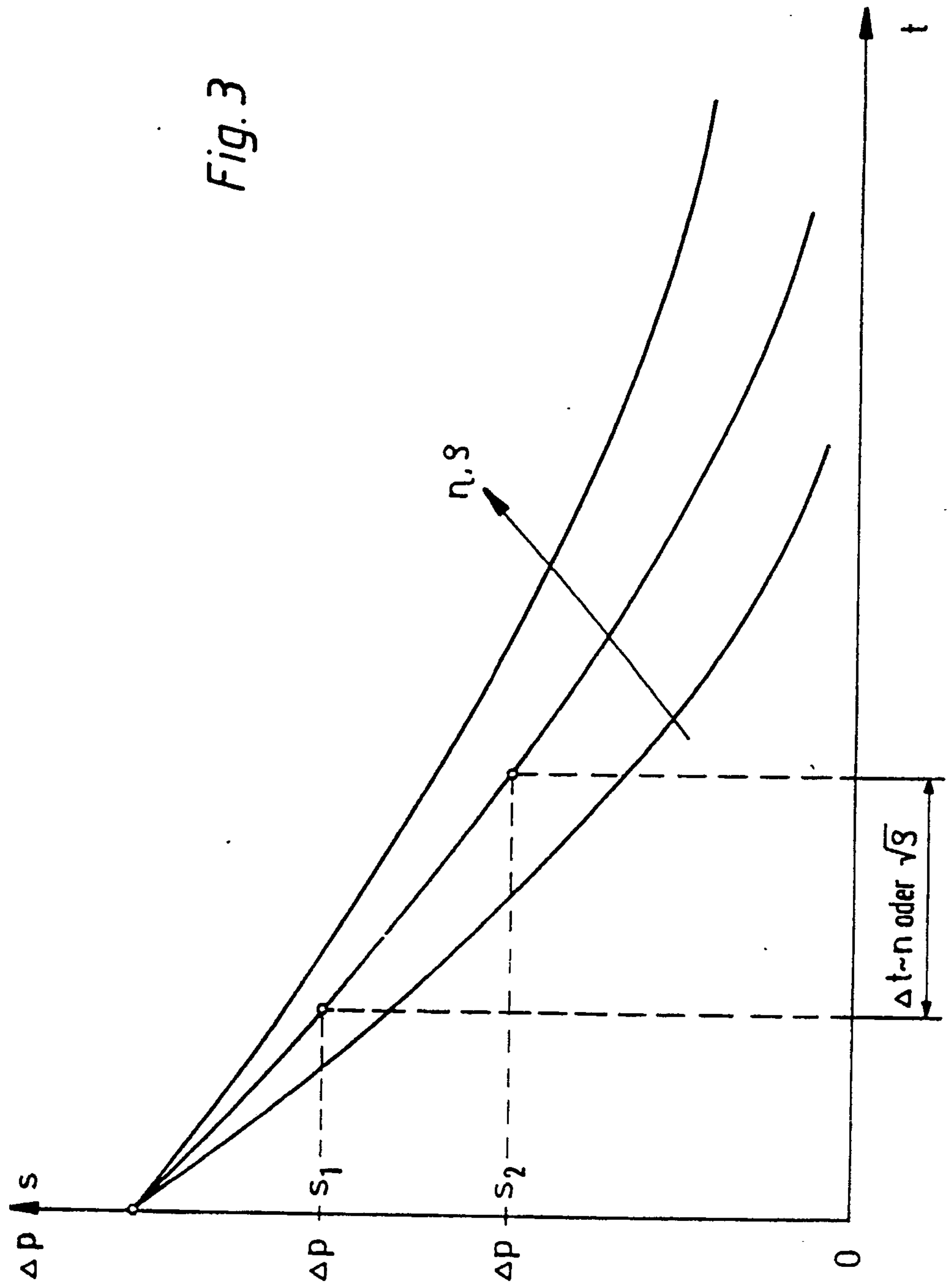


Fig. 3